# FASCICULE DE BREVET EUROPEEN

(11) **EP 0 479 633 B1**
(45) Date de publication et mention de la délivrance du brevet: **21.12.1994**
(21) Numéro de dépôt: 91402432.8
(22) Date de dépôt: 13.09.1991
(51) Int. Cl.: G01N 33/00

(54) **Procédé et dispositif de fourniture de gaz à un analyseur à très haute sensibilité**
Verfahren und Vorrichtung zur Lieferung von einem Gas an einen sehr empfindlichen Analysator
Method and apparatus for supplying gas to an analyser with a very high sensitivity

(30) Priorité: 02.10.1990 FR 9012101
(43) Date de publication de la demande: 08.04.1992
(73) Titulaire: L'AIR LIQUIDE, SOCIETE ANONYME POUR L'ETUDE ET L'EXPLOITATION DES PROCEDES GEORGES CLAUDE, 75321 Paris Cédex 07 (FR)
(72) Inventeur: Molozay, Maurice, F-78320 Le Mesnil Saint Denis (FR); Loiseau, Gérard, F-78390 Bois d'Arcy (FR); Ronge, Catherine, F-75013 Paris (FR)
(74) Mandataire: Le Moenner, Gabriel

(56) Documents cités:
- EP-A- 0 370 151
- EP-A- 0 370 871
- FR-A- 2 093 653
- US-A- 4 063 446
- US-A- 4 436 699

## Description

La présente invention concerne les procédés de fourniture de gaz à un analyseur, à très haute sensibilité, de traces de gaz résiduels dans un gaz pur (appelées par la suite "traces""), du type comprenant les étapes de fourniture séquentielle à un tronçon de ligne d'entrée à l'analyseur d'un débit déterminé:
a) d'un gaz à analyser, via une première ligne,
b) d'un gaz pur élaboré par épuration du gaz à analyser, via une deuxième ligne,
c) d'au moins un gaz de calibration, dilué par le gaz pur, contenant initialement un niveau de traces de gaz résiduels plus important que le gaz à analyser, via une troisième ligne,

les parties aval des deux premières lignes se raccordant au tronçon de ligne d'entrée, la partie aval de la troisième ligne se raccordant au tronçon ou sur la deuxième ligne.

Un procédé de ce type est décrit dans le document EP-A-370.871.

La mise en oeuvre d'analyseurs à très haute sensibilité de traces ou impuretés, notamment du type hygromètre ultrasensible ou spectroscope à infrarouge à transformée de Fourier (FTIR), capables de détecter et d'analyser des traces de gaz à des niveaux inférieurs au ppb et utilisés par exemple pour les gaz de très haute pureté (azote, argon) pour l'industrie des semi-conducteurs à très haute intégration (VLSI), implique de pouvoir fournir séquentiellement à l'analyseur, à un débit déterminé, le gaz à analyser, un gaz dit "zéro", obtenu par épuration du gaz à analyser et qualifiant le zéro de l'analyseur, et un gaz de calibration, obtenu par une forte dilution d'un gaz contenant initialement une quantité déterminée et importante de traces, de façon à calibrer la plage de lecture de l'analyseur.

Les procédés et dispositifs connus mettent en oeuvre trois lignes pourvues chacune d'un régulateur de débit et d'une vanne d'arrêt pour isoler les lignes non intéressées par une des phases de fourniture de gaz à l'analyseur. Ces procédés présentent deux inconvénients majeurs dans la mesure où les régulateurs de débit sont des composants polluants susceptibles d'ajouter des traces ou impuretés non contrôlées aux différentes lignes de gaz, et où, dans les périodes où les lignes sont isolées, le gaz dans ces lignes est susceptible d'être rapidement pollué par simple adsorption ou désorption des parois des tubes.

Pour réduire sensiblement les inconvénients ci-dessus, il a été proposé, dans le document sus-mentionné, un procédé et un dispositif, mettant en oeuvre deux lignes principales, la première alimentée par le gaz à analyser, la seconde par un gaz dit "zéro" (qualifiant le zero de l'analyseur). Le troisième type de ligne (calibration) vient se raccorder en sa partie aval, sur la deuxième ligne, en amont du point où cette deuxième ligne se raccorde au tronçon d'alimentation de l'analyseur.

Chacune de ces lignes est munie d'une perte de charge statique, moins polluante qu'un régulateur de débit, et en sa partie aval, d'un moyen d'isolation, permettant l'isolation de la ligne, avec balayage de la partie amont de la ligne.

Cette approche permet de réduire les longueurs de tubes isolées et non balayées mais pas de les supprimer totalement. Par ailleurs, la pression d'alimentation dans les première et seconde lignes peut varier dans une large plage, de sorte que le procédé et le dispositif proposés présentent des risques de dérive importante dans le temps ne permettant pas de garantir la fourniture, à l'analyseur, d'un débit déterminé, et pouvant donc fausser la précision des mesures.

La présente invention a pour objet de proposer un procédé de fourniture de gaz à un analyseur permettant d'éviter sur le trajet des différents gaz tout composant polluant, de maintenir en balayage permanent toutes les lignes du système, d'assurer une régulation des débits précise et peu susceptible de dérive dans le temps, et donc de garantir une qualité d'analyse fiable et notablement améliorée.

Pour ce faire, selon une caractéristique de l'invention, les parties amont des trois lignes sont chacune parcourues en permanence par le gaz correspondant à un débit constant, en ce qu'on prévoit, dans une partie intermédiaire de chaque ligne, une dérivation de décharge munie d'un régulateur de débit commandable, le débit constant dans les première et deuxième lignes étant supérieur au débit déterminé pour l'analyseur, et en ce qu'on commande, pour chaque phase a) et b) sus-mentionnée les régulateurs de débit de façon qu'une première partie du débit en excès dans la ligne correspondant à une phase soit évacuée par la dérivation de décharge associée et que des deuxième et troisième parties du débit en excès dudit gaz soient recirculées dans chacune des parties aval des deux autres lignes dont la totalité du débit en excès propre et la deuxième ou troisième partie du débit recirculé sont évacuées par la dérivation de décharge correspondante.

Selon une caractéristique plus particulière de l'invention, le débit constant est assuré, dans chaque ligne, par une restriction calibrée fonctionnant en régime sonique, la pression en amont de la restriction calibrée étant mesurée pour piloter au moins le régulateur de débit de la dérivation de décharge associée.

La présente invention a pour autre objet de proposer un dispositif de fourniture de gaz à un analyseur pour la mise en oeuvre du procédé ci-dessus, du type comprenant:
- une source de gaz à analyser;
- une source de gaz de calibration;
- une première ligne, comportant un premier moyen régulateur de débit, reliant directement la source de gaz à analyser à un tronçon de ligne d'entrée de l'analyseur;
- une deuxième ligne reliée, en amont, à la source de gaz à analyser et comportant, en série, un épurateur de gaz et un deuxième moyen régulateur de débit, et en aval au tronçon de ligne d'entrée à l'analyseur;
- au moins une troisième ligne, comportant un troisième moyen régulateur de débit, reliée à une source de gaz de calibration contenant initialement une quantité de traces plus importante que le gaz à analyser et, en aval, à une partie aval de la deuxième ligne ou au tronçon de ligne d'entrée à l'analyseur;

et se caractérisant par le fait que chaque ligne comporte, dans une partie amont, une restriction calibrée formant col sonique et, dans une partie intermédiaire, une dérivation de décharge pourvue d'un régulateur de débit de décharge.

Selon une autre caractéristique de l'invention, chaque ligne comprend, immédiatement en amont de la restriction dans cette ligne, un capteur de pression qui est typiquement monté sur une dérivation de mesure de pression débouchant dans l'atmosphère par un orifice calibré.

Avec le procédé et le dispositif selon l'invention, chaque ligne est en permanence intégralement balayée par un débit de gaz, en amont par le gaz circulant dans la ligne et, en aval et en phase non opérative, par une partie de gaz recirculé en provenance d'une autre ligne. Dans la partie active de chaque ligne, on ne trouve qu'un orifice ou une restriction calibrée, naturellement peu polluante et rendue ici sensiblement non susceptible d'adjoindre des impuretés en raison du balayage permanent de la ligne. Les organes polluants, comme les capteurs de pression et les régulateurs de débit, sont disposés dans des dérivations de la ligne proprement dite, également balayées en permanence par un débit de gaz. Les restrictions calibrées dans les lignes fonctionnant en régime sonique, les débits de gaz dans les lignes, en aval des restrictions, ne dépendent que de la pression amont et peuvent donc être facilement calculés à partir de cette dernière, ce qui permet d'assurer un fonctionnement précis, peu variable dans le temps et corrigeable, du système selon l'invention.

D'autres caractéristiques et avantages de la présente invention ressortiront de la description suivante de modes de réalisation, donnés à titre illustratif mais nullement limitatif, faite en relation avec les dessins annexés, sur lesquels :
- la figure 1 est une vue schématique d'un dispositif pour la mise en oeuvre du procédé de fourniture de gaz selon l'invention, les écoulements de gaz figurés correspondant à une phase d'analyse du gaz à analyser, et
- la figure 2 est une vue schématique d'un autre mode de réalisation d'un dispositif selon l'invention.

Dans la description qui va suivre et sur les dessins, les éléments identiques ou analogues sont repérés par les mêmes chiffres de référence.

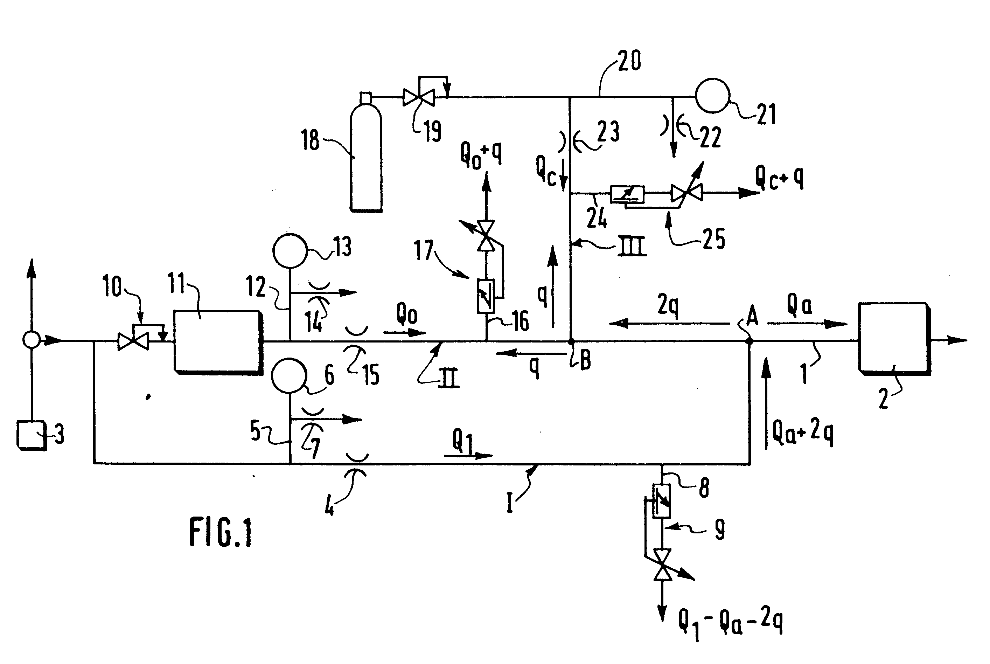

Le dispositif représenté sur la figure 1 comporte trois lignes I, II, III, typiquement en tubes d'acier inoxydable calmé et électro-poli, de fourniture sélective de gaz à un tronçon de ligne d'entrée 1 à un analyseur à très haute sensibilité 2, destiné à analyser des traces de gaz résiduels dans un gaz principal provenant d'une source de gaz ultra-pur 3, typiquement pour la mesure en continu de traces éventuelles dans un gaz élaboré in situ par une installation de fourniture de gaz 3, par exemple un générateur d'azote haute pureté (HPN).

Le gaz en provenance de la source 3 est fourni, à une pression généralement comprise entre 5 et 15 x 10⁵ Pa, à une première ligne I ou ligne d'analyse, et à une ligne II, toutes deux raccordées en aval, en A, au tronçon d'entrée 1 de l'analyseur 2.

La ligne I comporte une restriction 4, typiquement un orifice calibré, fonctionnant en régime sonique et, en amont de la restriction 4, une dérivation de mesure de pression 5 pourvue d'un capteur de pression 6 en amont d'une perte de charge calibrée 7 autorisant un très faible débit de fuite dans la dérivation 5. La ligne I comporte, en aval, une dérivation de décharge 8 pourvue d'un régulateur de débit commandable 9.

La ligne II, destinée à fournir à l'analyseur 2, le gaz "zéro" et le gaz de calibration dilué comporte, en amont, un régulateur de pression 10 et, en série, un épurateur 11, par exemple du type à adsorbant ou du type à épuration chimique dit "GETTER". En aval de l'épurateur 11, la ligne II comporte, comme la ligne I, une dérivation de mesure de pression 12, avec un capteur de pression 13 et un orifice calibré de décharge 14, une restriction calibrée 15 et une dérivation de décharge 16 pourvue d'un régulateur de débit commandable 17.

La ligne III établit une communication entre un réservoir de gaz de calibration 18, pourvu d'un mano-détendeur 19 et renfermant un gaz du type du gaz à analyser avec des mêmes traces mais à une teneur de l'ordre de 1000 à 10 000 fois supérieure. La ligne III communique avec la ligne II en un point B situé en aval de la dérivation 16 et en amont du point A. La ligne III, comme les autres lignes, comporte, successivement, une dérivation de mesure de pression 20 avec un capteur de pression 21 et un orifice calibré de décharge 22, une restriction calibrée 23 et une dérivation de décharge 24 pourvue d'un régulateur de débit commandable 25.

On va maintenant décrire, en relation avec la phase d'analyse de gaz, la mise en oeuvre du procédé selon l'invention. Les restrictions 4, 15 et 23 déterminent dans les lignes I, II, III des débits de gaz constants Q₁, Q_{O} et Q_{C}, respectivement. Dans la phase d'analyse, les lignes II et III ne sont pas mises en oeuvre, le gaz provenant de la source 3 étant directement adressé, via la ligne I, à l'analyseur 2. L'analyseur 2 fonctionne avec un débit d'amenée de gaz déterminé Qₐ, de l'ordre de 1 litre/minute. Le débit nominal Q₁, défini par la restriction 4, est déterminé de façon à être largement en excès par rapport au débit déterminé Qₐ, en l'occurrence Qₐ + 2q, q étant un débit de balayage choisi arbitrairement de l'ordre de 0,2 litre/minute. Dans cette phase d'analyse, le régulateur 9 dans la dérivation de décharge 8 de la ligne I est réglé de façon à laisser s'échapper un débit de fuite Q₁ - Qₐ - 2q. Au point A, le débit (Qₐ + 2q) en aval de la ligne I se divise en un débit Qₐ dans le tronçon d'entrée 1 et un débit 2q remontant dans le tronçon aval AB de la ligne II. Au point B, le débit de recirculation 2q se divise en un débit q remontant la ligne II et un débit q remontant la ligne III. Selon le procédé de l'invention, le régulateur de débit 17 dans la dérivation de décharge 16 de la ligne II est réglé de façon à évacuer de la ligne II le débit nominal Q_{O} délivré par la restriction 15 et le débit q de recirculation sus-mentionné. De façon similaire, le régulateur de débit 25 de la ligne III est réglé de façon à évacuer de cette ligne III la totalité du débit nominal Q_{c} délivré par la restriction 23 et le débit de recirculation q. Comme on le voit, toutes les portions de circuiterie actives du dispositif selon l'invention sont au même moment balayées par un débit de gaz conformément à l'objet de l'invention.

Le fonctionnement est similaire lors des autres phases de fourniture de gaz à l'analyseur. Ainsi, lors de la fourniture du gaz "zéro" par la ligne II, le régulateur 17 de la ligne II dérive un débit Q_{O} - Qₐ - 2q, le régulateur 25 de la ligne III dérivant le même débit Q_{c} + q que précédemment et le régulateur 9 de la ligne I dérivant de cette dernière un débit Q₁ + q.

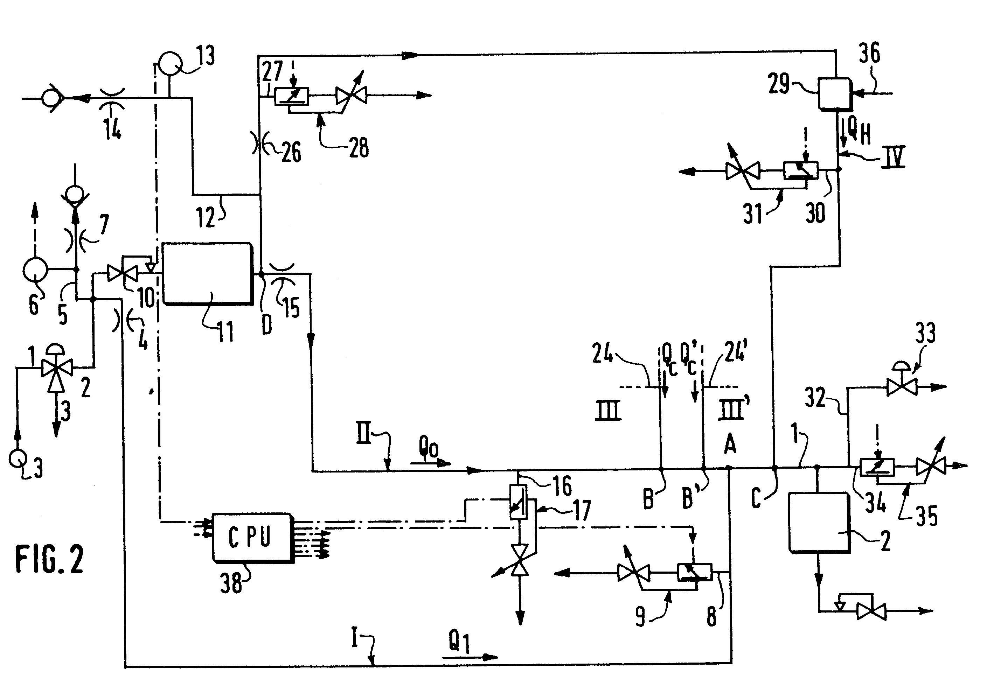

Dans le mode de réalisation de la figure 2, on retrouve les éléments de la circuiterie de la figure 1 avec ici plusieurs lignes de calibration dont une ligne III′ analogue à la ligne III précédemment décrite, avec une source de gaz de calibration différente du réservoir 18, et une ligne IV de calibration de traces de gaz en phase liquide, par exemple H₂O partant d'un point D de la ligne II entre l'épurateur 11 et la calibration 15 et rejoignant le tronçon d'entrée 1 à l'analyseur 2 en un point C en aval du point A de jonction de la ligne I. La ligne IV comporte, comme les lignes I à III, une calibration amont 26 suivie d'une première dérivation de décharge 27 munie d'un régulateur de débit 28 permettant d'assurer à un humidificateur 29 un débit de balayage constant précis, puis une seconde dérivation de décharge 30 munie d'un régulateur de débit 31. L'humidificateur 29, par exemple du type perméateur, est alimenté, en 36, par une source de liquide sous pression. Le capteur de pression 13 est commun aux lignes II et IV. Par rapport au mode de réalisation de la figure 1, le tronçon d'entrée 1 à l'analyseur 2 est prolongé en aval par une première dérivation de décharge 32 munie d'une vanne d'arrêt 33 et par une deuxième dérivation de décharge 34 munie d'un régulateur de débit 35.

Pour assurer une qualité d'analyse particulièrement fiable et indépendante des variations de pression et de température du gaz à analyser, le dispositif selon l'invention peut avantageusement, comme représenté sur la figure 2, être automatisé, les capteurs de pression 6, 13 et 21 fournissant en continu à une unité centrale de commande 38, typiquement du type à micro-processeur, des signaux de pression qui, comparés avec des valeurs de référence en mémoire dans l'unité centrale 38, permettent à cette dernière d'adresser aux différents régulateurs de débit 9, 17, 25, 28, 31, 35 des signaux de commande pour ajuster l'ouverture de ces derniers au débit réel circulant dans les lignes et déduit des pressions mesurées par les capteurs de pression.

## Revendications

1. Procédé de fourniture de gaz à un analyseur de traces à très haute sensibilité (2), comprenant les étapes de fourniture séquentielle à un tronçon de ligne d'entrée (1) à l'analyseur (2) d'un débit déterminé (Qₐ) :
a) d'un gaz à analyser, via une première ligne (I), raccordée en sa partie aval au tronçon, en un premier point (A);
b) d'un gaz pur élaboré par épuration du gaz à analyser, via une deuxième ligne (II), raccordée en sa partie aval au tronçon, en ledit premier point (A);
c) d'au moins un gaz de calibration, dilué par le gaz pur, contenant initialement une quantité de traces plus importante que le gaz à analyser, via au moins une troisième ligne (III, III′, IV), raccordée en sa partie aval à la deuxième ligne (II) en un second point (B, B') situé en amont du premier point (A), ou directement sur le tronçon en un troisième point (C) situé en aval du premier point (A);
caractérisé en ce que les parties amont des trois lignes (I; II; III ; III ′; IV) sont chacune parcourues en permanence par le gaz correspondant à un débit constant, en ce qu'on prévoit, dans une partie intermédiaire de chaque ligne, une dérivation de décharge (8, 16, 24) munie d'un régulateur de débit commandable (9, 17, 25), le débit constant (Q₁, Q₀) dans les première (I) et deuxième (II) lignes étant supérieur au débit déterminé (Qₐ), et en ce qu'on commande, pour chaque phase a) et b) les régulateurs de débit de façon qu'une première partie du débit en excès (Q₁, Q₀) du gaz dans la ligne (I, II) correspondant à une de ces phases soit évacuée par la dérivation de décharge associée (8 ; 16) et que des deuxième et troisième parties (q) du débit en excès dudit gaz soient recirculées dans chacune des parties aval des deux autres lignes dont la totalité du débit en excès propre et la deuxième ou troisième partie du débit recirculé sont évacuées par la dérivation de décharge correspondante.

2. Procédé selon la revendication 1, caractérisé en ce que le débit constant (Q₁, Q₀, Q_{c}) dans chaque ligne (I; II; III; III′; IV) est assuré par une restriction calibrée fonctionnant en régime sonique (4, 15, 23) et en ce qu'on mesure (6, 13, 21) la pression en amont de la restriction calibrée pour piloter au moins le régulateur de débit (9, 17, 25) de la dérivation de décharge associée (8, 16, 24).

3. Procédé selon la revendication 2, caractérisé en ce que la pression est mesurée dans une dérivation de mesure de pression (5, 12, 20) parcourue en permanence par un faible débit de fuite.

4. Dispositif de fourniture de gaz à un analyseur de traces à très haute sensibilité (2) pour la mise en oeuvre du procédé selon l'une des revendications 1 à 3, comprenant:
- une source de gaz à analyser (3);
- une première ligne (I), comportant un premier moyen régulateur de débit, reliant directement la source du gaz à analyser (3) à un tronçon de ligne d'entrée (1) à l'analyseur (2);
- une deuxième ligne (II) reliée, en amont, à la source de gaz à analyser (3) et comportant, en série, un épurateur de gaz (11) et un deuxième moyen régulateur de débit, et en aval au tronçon de ligne d'entrée (1) à l'analyseur (2);
- au moins une troisième ligne (III; III'; IV), comportant un troisième moyen régulateur de débit, reliée à une source de gaz de calibration (18, 19) contenant une quantité de traces plus importante que le gaz à analyser, et, en aval, à une partie aval (B ; B′) de la deuxième ligne (II) ou au tronçon de ligne d'entrée à l'analyseur (C);
caractérisé en ce que chaque ligne (I, II, III) comporte, dans une partie amont, une restriction calibrée formant col sonique (4 ; 15 ; 23 ; 26) et, dans une partie intermédiaire, une dérivation de décharge (8; 16 ; 24; 24'; 30) pourvue d'un régulateur de débit de décharge (9; 17 ; 25; 31).

5. Dispositif selon la revendication 4, caractérisé en ce que chaque régulateur de débit de décharge (9; 17 ; 25 ; 31) est actionnable entre au moins un premier état, où il évacue une partie du débit nominal (Q₁, Q₀, Q_{c}, Qᵤ), délivré par la restriction (4, 15, 23, 26) de la ligne correspondante, et un deuxième état où il évacue la totalité du débit nominal augmentée d'un débit (q) recirculé en provenance d'une autre ligne.

6. Dispositif selon la revendication 4 ou la revendication 5, caractérisé en ce que chaque ligne (I; II; III; III′; IV) comprend, immédiatement en amont de la restriction (4,15,23), un capteur de pression (6, 13, 21).

7. Dispositif selon la revendication 6, caractérisé en ce que le capteur de pression (6, 13, 21) est monté sur une dérivation de mesure de pression (5, 12, 20) débouchant dans l'atmosphère par un orifice calibré (7, 14, 22).

8. Dispositif selon l'une des revendications 4 à 7, caractérisé en ce que le tronçon de ligne d'entrée (1) communique, en aval, avec une conduite de décharge (34) pourvue d'un régulateur de débit de décharge (35).

9. Dispositif selon l'une des revendications 4 à 8, caractérisé en ce qu'il comporte au moins deux troisième lignes (III; III′; IV).

10. Dispositif selon la revendication 9, caractérisé en ce qu'au moins une troisième ligne (IV) est reliée, en amont, à la deuxième ligne (II), entre l'épurateur (11) et la restriction calibrée (15) de cette deuxième ligne, et en aval, au tronçon de ligne d'entrée (1) et comprend, en amont de la conduite de décharge (30), un générateur de traces balayé à débit constant (29).

11. Dispositif selon la revendication 10, caractérisé en ce que le générateur de traces (29) introduit dans la troisième ligne (IV) des traces à partir d'une source de liquide (36).

## Patentansprüche

1. Verfahren zur Lieferung von Gas an einen sehr empfindlichen Spurenanalysator (2), mit folgenden Schritten der sequentiellen Lieferung an einen Abschnitt einer Eingangsleitung (1) zu dem Analysator (2) mit einem bestimmten Durchfluß:
a) eines zu analysierenden Gases über eine erste Leitung (I), die in ihrem stromabseitigen Teil mit dem Abschnitt in einem ersten Punkt (A) verbunden ist;
b) eines durch Reinigen des zu analysierenden Gases gewonnenen, reinen Gases über eine zweite Leitung (II), die in ihrem stromabseitigen Teil mit dem Abschnitt in dem ersten Punkt (A) verbunden ist;
c) mindestens eines Kalibriergases, das mit dem reinen Gas verdünnt ist und anfänglich eine größere Menge an Spuren als das zu analysierende Gas enthält, über mindestens eine dritte Leitung (III, III′, IV), die in ihrem stromabseitigen Teil mit der zweiten Leitung (II) in einem zweiten Punkt (B, B′), der sich stromauf von dem ersten Punkt (A) befindet oder direkt mit dem Abschnitt in einem dritten Punkt (C), der sich stromab von dem ersten Punkt (A) befindet, verbunden ist; **dadurch gekennzeichnet** daß die stromaufseitigen Teile der drei Leitungen (I; II; III; III′; IV) jeweils ständig mit einem konstanten Durchfluß von dem Gas durchströmt werden; daß man in einem Zwischenteil jeder Leitung eine Auslaßableitung (8, 16, 24) vorsieht, die mit einem steuerbaren Durchflußregler (9, 17, 25) versehen ist, wobei der konstante Durchfluß (Q₁, Q₀) in der ersten (I) und zweiten (II) Leitung größer ist als der bestimmte Durchfluß (Qₐ); und daß man für jede Phase a) und b) die Durchflußregler derart steuert, daß ein erster Teil des überschüssigen Durchflusses (Q₁, Q₀) des Gases in des Leitung (I, II), der einer ihrer Phasen entspricht, durch die zugeordnete Auslaßableitung (8; 16) evakuiert wird und daß zweite und dritte Teile (q) des überschüssigen Durchflusses des Gases in den Kreislauf in jedem der stromabseitigen Teile der beiden anderen Leitungen zurückgeführt werden, wovon die Gesamtheit des eigenen überschüssigen Durchflusses und der zweite und dritte Teil des in den Kreislauf zurückgeführten Durchflusses durch die entsprechende Auslaßableitung evakuiert werden.

2. Verfahren nach Anspruch 1, **dadurch gekennzeichnet**, daß der konstante Durchfluß (Q₁, Q₀, Q_{c}) in jeder Leitung (I; II; III; III′; IV) durch eine kalibrierte Verengung sichergestellt wird, die im Schallbereich arbeitet (4, 15, 23), und daß man den Druck stromauf von der kalibrierten Verengung mißt (6, 13, 21), um zumindest den Durchflußregler (9, 17, 25) der zugeordneten Auslaßableitung (8, 16, 24) zu steuern.

3. Verfahren nach Anspruch 2, **dadurch gekennzeichnet**, daß der Druck in einer Druckmeßableitung (5, 12, 20) gemessen wird, die ständig von einem schwachen Leckdurchfluß durchströmt wird.

4. Vorrichtung zur Lieferung von Gas an einen sehr empfindlichen Spurenanalysator (2) zur Durchführung des Verfahrens nach einem der Ansprüche 1 bis 3, welche aufweist:
- eine Quelle des zu analysierenden Gases (3);
- eine erste Leitung (I), die eine erste Durchflußreglereinrichtung aufweist und die Quelle des zu analysierenden Gases (3) direkt mit einem Abschnitt des Eingangsleitung (1) zu dem Analysator (2) verbindet;
- eine zweite Leitung (II), die stromaufseitig mit der Quelle des zu analysierenden Gases (3) verbunden ist und in Serie einen Gasreiniger (11) und eine zweite Durchflußreglereinrichtung aufweist und stromabseitig mit dem Abschnitt der Eingangsleitung (1) zu dem Analysator (2) verbunden ist;
- mindestens eine dritte, eine dritte Durchflußreglereinrichtung aufweisende Leitung (III; III′; IV), die mit einer Kalibriergasquelle (18, 19) verbunden ist, die eine größere Menge an Spuren enthält als das zu analysierende Gas, und stromabseitig mit einem stromabseitigen Teil (B; B′) der zweiten Leitung (II) oder mit dem Abschnitt der Eingangsleitung zu dem Analysator (C) verbunden ist; **dadurch gekennzeichnet**, daß jede Leitung (I; II; III) in einem stromaufseitigen Teil eine einen Schallhals (4; 15; 23; 26) bildende kalibrierte Verengung aufweist und in einem Zwischenteil eine Auslaßableitung (8; 16; 24; 24′; 30) aufweist, die mit einem Auslaßdurchflußregeler (9; 17; 25; 31) versehen ist.

5. Vorrichtung nach Anspruch 4, **dadurch gekennzeichnet**, daß jeder Auslaßdurchflußregler (9; 17, 25; 31) schaltbar ist zwischen mindestens einem ersten Zustand, bei dem er einen Teil des nominalen Durchflusses (Q₁, Q₀, Q_{c}, Qᵤ) evakuiert, der durch die Verengung (4, 15, 23, 26) der entsprechenden Leitung geliefert wird, und einem zweiten Zustand, bei dem er die Gesamtheit des nominalen Durchflusses evakuiert, der um einen aus einer anderen Leitung stammenden, zurückgeführten Durchfluß (q) erhöht ist.

6. Vorrichtung nach Anspruch 4 oder 5, **dadurch gekennzeichnet,** daß jede Leitung (I; II; III; III′; IV) unmittelbar stromauf von der Verengung (4, 15, 23) einen Drucksensor (6, 13, 21) aufweist.

7. Vorrichtung nach Anspruch 6, **dadurch gekennzeichnet,** daß der Drucksensor (6, 13, 21) an einer Druckmeßableitung (5, 12, 20) montiert ist, die über eine kalibrierte Öffnung (7, 14, 22) in die Atmosphäre mündet.

8. Vorrichtung nach einem der Ansprüche 4 bis 7, **dadurch gekennzeichnet,** daß der Abschnitt der Eingangsleitung (1) stromabseitig mit einer Auslaßleitung (34) kommuniziert, die mit einem Auslaßdurchflußregler (35) versehen ist.

9. Vorrichtung nach einem der Ansprüche 4 bis 8, **dadurch gekennzeichnet,** daß sie mindestens zwei dritte Leitungen (III; III′; IV) aufweist.

10. Vorrichtung nach Anspruch 9, **dadurch gekennzeichnet,** daß mindestens eine dritte Leitung (IV) stromaufseitig mit der zweiten Leitung (II) zwischen dem Reiniger (11) und der kalibrierten Verengung (15) dieser zweiten Leitung verbunden ist, und stromabseitig mit dem Abschnitt der Eingangsleitung (1) verbunden ist und stromaufseitig von der Auslaßleitung (30) einen getasteten Spurengenerator (29) mit konstantem Durchfluß aufweist.

11. Vorrichtung nach Anspruch 10, **dadurch gekennzeichnet**, daß der Spurengenerator (29) in die dritte Leitung (IV) Spuren von einer Flüssigkeitsquelle (36) einführt.

## Claims

1. Method for supplying gas to a very high sensitivity trace analyser (2), comprising the stages of sequential supply to a section of feed line (1) to the analyser (2) at a given flow rate Qₐ:
a) of a gas to be analysed, via a first line (I) connected at its downstream end to the section, at a first point (A);
b) of a pure gas produced by purifying the gas to be analysed, via a second line (II), connected at its downstream end to the section, at the said first point (A);
c) of at least one calibration gas, diluted by the pure gas, initially containing a larger quantity of traces than the gas to be analysed, via at least one third line (III, III′, IV), connected at its downstream end to the second line (II) at a second point (B, B′) situated upstream of the first point (A), or directly to the section at a third point (C) situated downstream of the first point (A) ;
characterised in that the upstream parts of the three lines (I; II; III; III′; IV) each have the corresponding gas passing through them continuously at a constant flow rate, in that there is provided, in an intermediate part of each line, a discharge branch (8, 16, 24) equipped with a controllable flow regulator (9, 17, 25), the constant flow rate (Q₁, Q₀) in the first (I) and second (II) lines being greater than the given flow rate (Qₐ), and in that the flow regulators are controlled, for each phase a) and b), so that a first part of the excess flow (Q₁, Q₀) of the gas in the line (I, II) corresponding to one of these phases is discharged by the associated discharge branch (8; 16) and so that the second and third parts (q) of the excess flow of the said gas are recirculated in each of the downstream parts of the other two lines, the whole of whose total excess flow proper and the second or third part of whose recirculated flow are discharged by the corresponding discharge branch.

2. Method according to Claim 1, characterised in that the constant flow rate (Q₁, Q₀, Q_{c}) in each line (I; II; III; III′; IV) is ensured by a calibrated restriction functioning sonically (4, 15, 23) and in that the pressure upstream of the calibrated restriction is measured (6, 13, 21) in order to control at least the flow regulator (9, 17, 25) of the associated discharge branch (8, 16, 24).

3. Method according to Claim 2, characterised in that the pressure is measured in a pressure-measuring branch (5, 12, 20) through which a small quantity of gas escapes continuously.

4. Device for supplying gas to a very high sensitivity trace analyser (2) for the implementation of the method according to one of Claims 1 to 3, comprising;
- a source of gas to be analysed (3);
- a first line (I), including a first flow-regulation means, connecting the source of the gas to be analysed (3) directly to a section of feed line (1) to the analyser (2);
- a second line (II) connected, upstream, to the source of gas to be analysed (3) and including, in series, a gas purifier (11) and a second means for regulating the flow, and, downstream, to the section of feed line (1) to the analyser (2) ;
- at least one third line (III; III′; IV), including a third flow-regulation means, connected to a source of calibration gas (18, 19) containing a larger quantity of traces than the gas to be analysed and, downstream, to a downstream part (B; B′) of the second line (II) or to the section of feed line to the analyser (C);
characterised in that each line (I, II, III) includes, in an upstream part, a calibrated restriction forming a sonic neck (4; 15; 23; 26) and, in an intermediate part, a discharge branch (18; 16; 24; 24′; 30) provided with a discharge flow rate regulator (9; 17; 25; 31).

5. Device according to Claim 4, characterised in that each discharge flow rate regulator (9; 17; 25, 31) can be actuated between at least one first state in which it discharges part of the nominal flow (Q₁, Q₀, Q_{c}, Qᵤ), delivered by the restriction (4, 15, 23, 26) of the corresponding line, and a second state in which it discharges the whole of the nominal flow increased by a recirculated flow (q) from another line.

6. Device according to Claim 4 or Claim 5, characterised in that each line (I; II; III; III′; IV) comprises, immediately upstream of the restriction (4, 15, 23), a pressure sensor (6, 13, 21).

7. Device according to Claim 6, characterised in that the pressure sensor (6, 13, 21) is mounted on a pressure-measuring branch (5, 12, 20) opening out to atmosphere via a calibrated orifice (7, 14, 22).

8. Device according to one of Claims 4 to 7, characterised in that the section of feed line (1) communicates, downstream, with a discharge pipe (34) provided with a discharge flow rate regulator (35).

9. Device according to one of Claims 4 to 8, characterised in that it comprises at least two third lines (III; III′; IV).

10. Device according to Claim 9, characterised in that at least one third line (IV) is connected, upstream, to the second line (II), between the purifier (11) and the calibrated restriction (15) of this second line, and, downstream, to the section of feed line (1), and comprises, upstream of the discharge pipe (30), a trace generatar scavenged at a constant flow rate (29).

11. Device according to Claim 10, characterised in that the trace generator (29) introduces traces from a source of liquid (36) into the third line (IV).
